# EUROPEAN PATENT APPLICATION

(11) **EP 4 772 615 A1**
(43) Date of publication of application: **08.07.2026**
(21) Application number: 24859404.6
(22) Date of filing: 07.08.2024
(51) Int. Cl.: C12N 7/01, A61K 35/761, A61P 35/00, C12N 15/35

(54) **RECOMBINANT ONCOLYTIC ADENOVIRUS**

(30) Priority: 28.08.2023 JP 2023138116
(71) Applicant: The University of Osaka, Osaka 565-0871 (JP)
(72) Inventor: MIZUGUCHI, Hiroyuki, Suita-shi, Osaka 565-0871 (JP); SAKURAI, Fuminori, Suita-shi, Osaka 565-0871 (JP)
(74) Representative: Hertin und Partner Rechts- und Patentanwälte PartG mbB
(86) International application number: PCT/JP2024/028319
(87) International publication number: WO 2025/047364

(57) **Abstract**

Provided is a recombinant oncolytic adenovirus (Ad) including, as a backbone, an Ad serotype except an adenovirus serotype 5 (Ad serotype 5), the recombinant oncolytic Ad achieving a high virus yield and being capable of exhibiting a sufficient antitumor action. Also provided is a pharmaceutical composition including the recombinant oncolytic Ad as an active ingredient. The recombinant oncolytic Ad is characterized in that: part or the entirety of the E1 gene of the Ad serotype 5 is incorporated into the E1 region of the genome of the Ad serotype except the Ad serotype 5; and part or the entirety of the E4 gene of the Ad serotype 5 is incorporated into the E4 region of the genome of the Ad serotype except the Ad serotype 5.

## Description

### Technical Field

The present invention relates to an oncolytic adenovirus including, as a backbone, an adenovirus serotype except an adenovirus serotype 5, and a pharmaceutical composition including the virus as an active ingredient.

The present application claims priority from Japanese Patent Application No. 2023-138116, which is incorporated herein by reference.

### Background Art

An oncolytic virus that specifically infects a cancer cell to kill the cancer cell has been attracting great attention as a new anticancer agent. Oncolytic viruses formed of 10 or more kinds of various viruses have heretofore been developed, and an oncolytic adenovirus (Ad) is one of the oncolytic viruses whose clinical development has been advanced to the largest extent.

The structure of an Ad genome has been analyzed in detail, and two methods have been mainly developed for causing an Ad to exhibit a cancer cell-specific growth function. A first attempt is a method including applying specific mutation or deletion to the early gene of an Ad to expect a limited growth property based on a biological property specific to a cancer cell, and is, for example, a mutant Ad of a p53-binding region deletion type (obtained by deleting an E1B gene 55kD). The other method is a method including expressing an E1 gene essential for the replication of an Ad with a tumor-specific promoter to cause virus replication in a tumor-specific manner, to thereby kill a cell. Currently, the latter method is used more frequently than the former. An antitumor effect exhibited by the oncolytic Ad has been considered to be largely attributable not only to a direct cytocidal effect along with the virus replication but also to stimulation by damage-associated molecular patterns (DAMPs) along with the killing of a cancer cell and the initiation of an immune system such as antiviral immunity. From the viewpoint of efficiently activating antitumor immunity, an attempt has been made to incorporate, for example, various cytokines and immunity-related genes that activate the immune system into the oncolytic Ad.

Meanwhile, there exist about 70 kinds of serotypes of Ads even when the serotypes are limited to human-derived serotypes, and Ads derived from various animal species except a human have been identified. Most of the oncolytic Ads that have heretofore been developed each include an Ad serotype 5 belonging to a subgroup C as a backbone. However, there has been a problem in that the expression of the infection receptor (coxsackievirus-adenovirus receptor; CAR) of the Ad serotype 5 is low in some cancer cells typified by a cancer cell having high malignancy, and hence the Ad serotype 5 hardly infects a CAR-negative cell and cannot efficiently infect the cell. In addition, most (90% or more) of adults each retain a neutralizing antibody against the Ad serotype 5 through natural infection, and hence a possibility that the therapeutic effect of the Ad is attenuated by the antibody has been pointed out.

There is a disclosure of a new oncolytic Ad serotype 35 in which attention is paid to an Ad serotype 35 belonging to a subgroup B (Patent Literature 1 and Non Patent Literature 1). In Non Patent Literature 1, there is a report of an oncolytic Ad serotype 35 obtained by incorporating a human telomerase reverse transcriptase (hTERT) promoter and the mutant E1 fragment of an Ad serotype 35 genome into the E1-deleted region of the Ad serotype 35 genome. The ratio of persons each retaining an antibody against the Ad serotype 35 is as low as about 20% or less, and hence it is conceivable that there is a low possibility that the therapeutic effect of the Ad is attenuated by the antibody. Further, it has been known that CD46 serving as the infection receptor of the Ad serotype 35 is expressed in all cells except an erythrocyte in a human, and is highly expressed particularly in a cancer cell having high malignancy. The Ad serotype 35 can infect a wide variety of cancer cells including, in particular, a CAR-negative cell, and is expected to be capable of avoiding an influence caused by an existing antibody. The oncolytic Ad serotype 35 can efficiently infect a wide variety of cancer species including a cancer cell having high malignancy, and hence a high therapeutic effect can be expected therefrom.

The development of an oncolytic Ad, which solves the problems of an oncolytic Ad serotype 5, and exhibits a sufficient virus yield and a sufficient antitumor action, has been desired.

### Citation List

### Patent Literature

[PTL 1] WO 2020/166727 A1

### Non Patent Literature

[NPL 1] Molecular Therapy: Oncolytics Vol. 20 March 2021, p. 399-409

### Summary of Invention

### Technical Problem

An object of the present invention is to provide a recombinant oncolytic Ad including, as a backbone, an Ad serotype except an Ad serotype 5, the recombinant oncolytic Ad achieving a high virus yield and being capable of exhibiting a sufficient antitumor action. Another object of the present invention is to provide a pharmaceutical composition including the oncolytic Ad as an active ingredient.

### Solution to Problem

With a view to solving the above-mentioned problems, the inventors of the present invention have made extensive investigations by paying attention to a region required for virus growth in an Ad genome, and as a result, have found that the above-mentioned problems can be solved by incorporating part or the entirety of a gene (E1 gene) encoding the E1 region of an Ad serotype 5, and part or the entirety of a gene (E4 gene) encoding the E4 region thereof into the E1 region and E4 region of the genome of an Ad serotype except the Ad serotype 5, respectively. Thus, the inventors have completed the present invention.

That is, the present invention includes the following.
1. A recombinant oncolytic Ad, including, as a backbone, an Ad serotype except an Ad serotype 5, wherein part or an entirety of an E1 gene of the Ad serotype 5 is incorporated into an E1 region of a genome of the Ad serotype except the Ad serotype 5, and wherein part or an entirety of an E4 gene of the Ad serotype 5 is incorporated into an E4 region of the genome of the Ad serotype except the Ad serotype 5.
2. The recombinant oncolytic Ad according to the above-mentioned item 1, wherein the part or the entirety of the E1 gene of the Ad serotype 5 includes at least a gene encoding an E1B55kD protein of the Ad serotype 5.
3. The recombinant oncolytic Ad according to the above-mentioned item 1 or 2, wherein the part or the entirety of the E4 gene of the Ad serotype 5 includes one or a plurality of kinds of genes selected from a gene encoding an open reading frame (ORF) 4 in an E4 protein of the Ad serotype 5, a gene encoding an ORF 6 therein, and a gene encoding an ORF 6/7 therein.
4. The recombinant oncolytic Ad according to any one of the above-mentioned items 1 to 3, wherein the recombinant oncolytic Ad further includes a tumor-specific promoter incorporated thereinto.
5. The recombinant oncolytic Ad according to the above-mentioned item 4, wherein the tumor-specific promoter is any promoter selected from a hTERT promoter, a survivin promoter, an α-fetoprotein promoter, a carcinoembryonic antigen (CEA) promoter, a prostate-specific antigen promoter, a midkine promoter, and a cyclooxygenase-2 (COX-2) promoter.
6. The recombinant oncolytic Ad according to any one of the above-mentioned items 1 to 5, further including a gene encoding a cell death induction-related protein or an immunostimulation-related protein.
7. The recombinant oncolytic Ad according to any one of the above-mentioned items 1 to 6, wherein the Ad serotype except the Ad serotype 5 is an Ad serotype 35.
8. A pharmaceutical composition for cancer treatment, including the recombinant oncolytic Ad of any one of the above-mentioned items 1 to 7 as an active ingredient.
9. A reagent for cancer treatment research, including the recombinant oncolytic Ad of any one of the above-mentioned items 1 to 7.
10. A method of producing the recombinant oncolytic Ad of any one of the above-mentioned items 1 to 6.
11. A method of improving a yield of a recombinant oncolytic Ad including, as a backbone, an Ad serotype except an Ad serotype 5, the method including: incorporating part or an entirety of an E1 gene of the Ad serotype 5 into an E1 region of a genome of the Ad serotype except the Ad serotype 5; and incorporating part or an entirety of an E4 gene of the Ad serotype 5 into an E4 region of the genome of the Ad serotype except the Ad serotype 5.

### Advantageous Effects of Invention

The recombinant oncolytic Ad of the present invention showed a physical titer and a biological titer higher than those of, for example, a related-art Ad serotype 35, and hence a high virus yield was recognized. Further, the recombinant oncolytic Ad had a high biological titer, and was hence able to secure a certain amount of viruses each having high activity. The Ad serotype except the Ad serotype 5 such as the Ad serotype 35 can eliminate an influence caused by an existing antibody because its anti-Ad antibody retention ratio in a human is extremely low as compared to that of the Ad serotype 5. In addition, CD46 serving as the receptor of the Ad serotype 35, which is expressed in substantially all human cells, is increased in expression particularly in a cancer cell, and hence its infection into the cancer cell can be enhanced. The recombinant oncolytic Ad of the present invention can sufficiently exhibit the antitumor action of an oncolytic Ad.

### Brief Description of Drawings

FIG. 1 is a view for illustrating an oncolytic Ad serotype 35 (OAd35), and a recombinant oncolytic Ad serotype 35 (OAd35-E1/E4) obtained by replacing part of the genome of the E1 region of the Ad and part of the genome of the E4 region thereof with part of the genome of the E1 region of an Ad5 and part of the genome of the E4 region thereof, respectively (Example 1).
FIG. 2 is a diagram for illustrating a plasmid for producing the OAd35-E1/E4 (Example 1).
FIG. 3 is a set of photographs showing the results of the recognition of the cytocidal actions of the OAd35 and the OAd35-E1/E4 on HCT116 (human colon cancer) cells, BxPC-3 (human pancreatic cancer) cells, H1299 (human non-small cell lung cancer) cells, and HEK293A (human embryonic kidney) cells by a crystal violet method (Experimental Example 2).
FIG. 4 is a set of graphs showing the results of the recognition of viabilities by a WST-8 assay method when the respective viruses OAd35 and OAd35-E1/E4 are added to each of the HCT116 cells, the BxPC-3 cells, the H1299 cells, and the HEK293A cells, and are cultured (Experimental Example 3).
FIG. 5 is a set of graphs showing the results of the recognition of the respective virus infection and growth potentials of the respective OAd35 and OAd35-E1/E4 by the measurement of virus genome copy numbers when the viruses are added to each of the H1299 cells, the HCT116 cells, the HEK293A cells, and the BxPC-3 cells, and are cultured (Experimental Example 4).
FIG. 6 is a graph showing the results of the evaluation of the virus infectivities of the OAd35 and the OAd35-E1/E4 by the ratio of a physical titer (VPs/mL) to a biological titer (PFUs/mL) (Experimental Example 5).
FIGS. 7 show the results of the recognition of the tumor growth-suppressing actions of the OAd35 and the OAd35-E1/E4. FIG. 7(A) is an illustration of an experimental protocol, and FIG. 7(B) is a graph showing the results (Experimental Example 6).
FIGS. 8 show the results of the recognition of the virus growth action of each of the OAd35 and the OAd35-E1/E4 in a tumor. FIG. 8(A) is an illustration of an experimental protocol, and FIG. 8(B) is a graph showing the results (Experimental Example 7).

### Description of Embodiments

The present invention is described below in detail. The scope of the present invention is not bound to such description, and modifications except the following examples may be appropriately performed to the extent that the gist of the present invention is not impaired. The present invention relates to a recombinant oncolytic Ad that achieves a high virus yield and can exhibit a sufficient antitumor action. An Ad comes in many serotypes. For example, there exist about 70 kinds of serotypes even when the serotypes are limited to human-derived serotypes, and Ads derived from various animal species except a human have been identified. Most of the oncolytic Ads that have heretofore been developed each include an Ad serotype 5 belonging to a subgroup C as a backbone. The term "Ad serotype except an Ad serotype 5" as used herein literally refers to an Ad serotype except the Ad serotype 5, and the Ad is not limited to a human-derived Ad, and may be an Ad derived from any other species such as a monkey. The Ad serotype except the Ad serotype 5 is preferably an Ad serotype that does not belong to the subgroup C, more preferably an Ad serotype belonging to a subgroup B, particularly preferably an Ad serotype 35. The term "recombinant Ad including, as a backbone, an Ad serotype except an Ad serotype 5" refers to an Ad mainly formed of the genome of the Ad serotype except the Ad serotype 5 defined above, the Ad being obtained by partially modifying the genome of the Ad serotype except the Ad serotype 5. Specifically, the term refers to a recombinant Ad characterized in that: part or the entirety of the E1 gene of the Ad serotype 5 is incorporated into the E1 region of the genome of the Ad serotype except the Ad serotype 5; and part or the entirety of the E4 gene of the Ad serotype 5 is incorporated into the E4 region of the genome of the Ad serotype except the Ad serotype 5.

The Ad genome is formed of a single double-stranded linear DNA, and encodes genes (E1A, E1B, E2A, E2B, E3, and E4) to be expressed at an early stage, and genes (L1 to L5) to be expressed at a later stage. The E1 genes of the Ad include the E1A gene and the E1B gene. An E1A protein encoded by the E1A gene activates the transcription of a group of genes (e.g., E1B, E2, and E4) required for the production of viruses that can infect host cells. An E1B protein encoded by the E1B gene helps the mRNA of each of the genes (L genes) to be expressed at the later stage accumulate in the cytoplasm of each of the infected host cells, and accelerates the replication of the viruses. It has been reported that in the growth of an Ad, an E1B55K protein of the Ad and an open reading frame (ORF) 6 protein in the E4 protein of the Ad cooperate with each other to be involved in, for example, the transportation of the mRNA of an Ad late gene (SILKE WEIGEL et al., JOURNAL OF VIROLOGY, Jan. 2000, p. 764-772). For example, when a nongrowing Ad serotype 5 grows in HEK293 cells, the E1B55K protein of the Ad5 and the ORF 6 protein of the E4 protein of the Ad5 expressed in the HEK293 cells form a composite to accelerate the transportation and replication of a viral gene. The composite of the E1B55K protein and the ORF 6 protein of the E4 protein needs to be a protein derived from Ads identical in type to each other.

In this description, the incorporation of "part or the entirety of the E1 gene of the Ad serotype 5" into the E1 region of the genome of the Ad serotype except the Ad serotype 5 literally means that the part or the entirety of the E1 gene of the Ad serotype 5 is incorporated into the E1 region of the genome of the Ad serotype except the Ad serotype 5. In particular, a region of the E1 gene of the Ad serotype 5 required for the activation of the transcription of a group of genes required for the production of an Ad only needs to be incorporated. Specifically, at least part or the entirety of the E1A gene of the Ad serotype 5, and/or part or the entirety of the E1B gene thereof only needs to be incorporated into the E1 region of the genome of the Ad serotype except the Ad serotype 5. More specifically, at least a gene (E1B55kD gene) encoding the E1B55kD protein of the Ad serotype 5 is desirably incorporated thereinto. In addition, in the present invention, mutation may be applied to the E1 gene essential for virus growth. For example, mutation may be applied to the sequence of the gene for avoiding the activation of innate immunity. Thus, a function, such as the optimization of a tumor-specific promoter (utilization of a hTERT or survivin promoter) or the retention of an adenovirus death protein (ADP) gene, is imparted, and hence a more excellent oncolytic Ad can be obtained.

In this description, the incorporation of "part or the entirety of the E4 gene of the Ad serotype 5" into the E4 region of the genome of the Ad serotype except the Ad serotype 5 literally means that the part or the entirety of the E4 gene of the Ad serotype 5 is incorporated into the E4 region of the genome of the Ad serotype except the Ad serotype 5. In particular, the E4 gene of the Ad serotype 5 required for the activation of the transcription of the group of genes required for the production of an Ad only needs to be incorporated. More specifically, one or a plurality of kinds of genes selected from a gene encoding at least an ORF 4 in the E4 protein of the Ad serotype 5, a gene encoding at least the ORF 6 therein, and a gene encoding at least an ORF 6/7 therein are desirably incorporated thereinto. In addition, in the present invention, mutation may be applied to the E4 gene of the Ad serotype 5 to be incorporated.

A recombinant oncolytic Ad serotype 35 of the present invention may be produced by the following method. First, a plasmid having the complete genome of the Ad serotype except the Ad serotype 5 is produced, and the E1 gene having incorporated thereinto the part or the entirety of the E1 gene of the Ad serotype 5 by the recombination of the plasmid is inserted into the genome of the Ad serotype except the Ad serotype 5. Thus, the recombinant Ad of the present invention can be produced.

In the recombinant oncolytic Ad of the present invention, the oncolytic Ad can be designed so as to grow in a cancer cell-specific manner by arranging the E1 genes (the E1A gene and the E1B gene), which are essential for the self-growth of the Ad, downstream of a tumor-specific promoter. The tumor-specific promoter only needs to be a promoter that functions in accordance with its corresponding tumor, and hence the promoter is not particularly limited. However, for example, a human telomerase reverse transcriptase (hTERT) promoter having high activity in each of many human cancer cells may be arranged. Other tumor-specific promoters, such as a survivin promoter, an α-fetoprotein (AFP) promoter, a carcinoembryonic antigen (CEA) promoter, a prostate-specific antigen (PSA) promoter, a DF3/MUC1 promoter, a midkine (MK) promoter, and a cyclooxygenase-2 (COX-2) promoter, may each also be used.

In addition to the insertion of the E1 gene, which has incorporated thereinto the part or the entirety of the E1 gene of the Ad serotype 5, downstream of the tumor-specific promoter, an E1 gene expression cassette, which is designed so as to express the E1B gene to an extent larger than a promoter intrinsic to a virus does, can be inserted into the E1-deleted region of the genome of the Ad serotype except the Ad serotype 5. The recombinant Ad of the present invention can be recovered by: treating a plasmid encoding the Ad genome having inserted thereinto the E1 gene expression cassette with a restriction enzyme to linearize the plasmid; and then introducing a gene into each of packaging cells. In addition to HEK293 cells, for example, H1299 cells, HeLa cells, or A549 cells may be used as the packaging cells.

The recombinant Ad of the present invention may further include a gene encoding a cell death induction-related protein or an immunostimulation-related protein because the Ad grows in a tumor-specific manner. Examples of the cell death induction-related protein and the immunostimulation-related protein include: a protein that can induce the apoptosis of target cells; and a protein that can enhance the functions of immune cells, such as cytotoxic T lymphocytes (CTLs) and natural killer cells.

The activity of the recombinant oncolytic Ad of the present invention can be evaluated by measuring its physical titer and biological titer. As a physical titer, the concentration of Ad particles including virus particles that are each free of infectivity may be determined by measuring the absorbance of the Ad at 260 nm (UV) in accordance with, for example, the method of Maizel et al. (Virology, 36: 115-125 (1968)). The method provides, as Ad particle concentrations, a vector particle (VP) and a particle titer (PT). The concentration of the virus particles per 1 mL may be represented in the unit of VP/mL or PT/mL. A plaque forming unit (PFU), an infectious unit (IFU), and a tissue culture infectious dose 50 (50% cytopathic endpoint, TCID₅₀) may be measured as biological titers. The magnitude of the physical titer relative to the biological titer may be represented as a ratio. As the number of viruses each having infectivity increases, the numerical value of the ratio reduces. A multiplicity of infection (MOI) represents the number of viruses with respect to one cell, and may be specifically represented as follows: MOI=PFUs/cell.

The physical titers and biological titers of an Ad serotype 5 from which its E1 region had been deleted and an Ad serving as an Ad serotype except the Ad serotype 5 such as an Ad, which was obtained by modifying part of the E4 gene of the Ad serotype 35 genome of an Ad serotype 35 with part of the E4 gene of an Ad serotype 5 genome, were measured. As a result, although the Ad obtained by modifying the E4 region of the Ad serotype 35 genome showed a physical titer and a biological titer comparable to those of the Ad serotype 5, the physical titer and biological titer of the Ad serotype 35 that was not modified were low. Further, the value (ratio) of the physical titer to the biological titer in the Ad serotype 35 that was not modified was extremely high. Accordingly, it was recognized that the degree of the production of viruses each having infectivity in the Ad serotype 35 was extremely low. Meanwhile, in the recombinant Ad serotype 35 of the present invention in which part or the entirety of the E1 gene of the Ad serotype 5 was incorporated into the E1 region of the Ad serotype 35 genome, and part or the entirety of the E4 gene of the Ad serotype 5 was incorporated into the E4 region of the Ad serotype 35 genome, its physical titer and biological titer, and the value (ratio) of the physical titer to the biological titer were comparable to those of the Ad serotype 5. Accordingly, it was recognized that the degree of the production of viruses each having infectivity was high. Again, for example, the Ad serotype 35 is excellent in that its infection into a cancer cell can be enhanced by using, as its receptor, CD46 that is increased in expression in the cancer cell. Further, high innate immunity inducibility exhibited by an Ad antibody provides an extremely large advantage because the antibody serves as an oncolytic virus intended for cancer treatment to lead to the enhancement of antitumor immunity.

The present invention covers a pharmaceutical composition including the recombinant oncolytic Ad of the present invention as an active ingredient. The pharmaceutical composition of the present invention may be used for cancer treatment. Further, the present invention covers a method of treating a cancer characterized by including administering the recombinant oncolytic Ad or the pharmaceutical composition to a cancer patient. The kind of the tumor (cancer) to be treated only needs to be a cancer in which the recombinant oncolytic Ad of the present invention can grow, and hence the tumor is not particularly limited. Examples thereof include a brain tumor, head and neck cancer, gastric cancer, colon cancer, lung cancer, liver cancer, prostate cancer, pancreatic cancer, esophageal cancer, bladder cancer, gallbladder cancer, bile duct cancer, breast cancer, uterine cancer, thyroid cancer, ovarian cancer, leukemia, lymphoma, sarcoma, and a mesenchymal tumor. Of those, breast cancer is preferred.

The recombinant oncolytic Ad of the present invention serving as an active ingredient may be used as it is because the Ad grows in a cancer-specific manner. Alternatively, the Ad may be used by being mixed with, for example, a known pharmaceutically acceptable carrier, such as an excipient, an extender, a binder, or a lubricant, or a known additive (including, for example, a buffering agent, an isotonizing agent, a chelating agent, a colorant, a preservative, a perfume, a flavoring agent, or a sweetener).

The pharmaceutical composition of the present invention may be applied as it is to an affected part, or may be introduced into a living organism (a cell or organ of interest) by any one of various known methods, such as: intratumoral, intravenous, intramuscular, intraperitoneal or subcutaneous injection; inhalation from a nasal cavity, an oral cavity, or a lung; oral administration; and intravascular administration with a catheter or the like.

The pharmaceutical composition of the present invention may be orally administrated or parenterally administered in accordance with a form, such as: an oral administration agent, such as a tablet, a capsule, a powder, a granule, a pill, a liquid formulation, or syrup; or a parenteral administration agent, such as an injection, an external preparation, a suppository, or an eye drop. Preferred examples thereof include: local injection into a tumor, a muscle, or an abdominal cavity; and injection into a vein.

The dose of the recombinant oncolytic Ad of the present invention serving as an active ingredient is appropriately selected in accordance with conditions, such as an administration route, an administration target, and the age, body weight, sex, and symptom of a patient. However, the dose thereof per day is desirably set to, for example, from about 10⁶ PFUs to about 10¹¹ PFUs, preferably from about 10⁹ PFUs to about 10¹¹ PFUs. The Ad may be administered once a day, or may be administered in several portions. Combined use of the recombinant oncolytic Ad of the present invention with a known anticancer agent or combined use thereof with, for example, radiotherapy is not prevented.

### Examples

The present invention is described in detail below by way of Examples and Experimental Examples for a better understanding of the present invention. Needless to say, however, the present invention is by no means limited to these Examples and the like.

### (Example 1) Production of Oncolytic Ad obtained by replacing each of E1 Region and E4 Region with Ad5

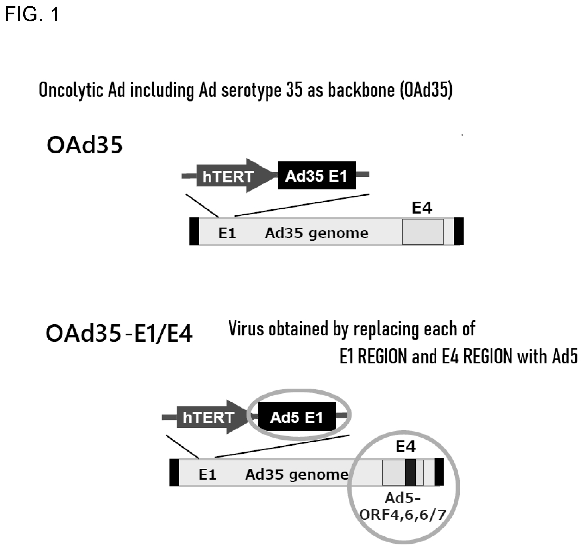

An oncolytic Ad was designed so as to grow in a cancer cell-specific manner by arranging E1 genes (an E1A gene and an E1B gene), which were essential for the self-growth of the Ad, downstream of a hTERT promoter serving as a tumor-specific promoter. In this Example, there was produced an oncolytic Ad (hereinafter also referred to as "OAd35-E1/E4") obtained by replacing part of the genome of the E1 region of an Ad serotype 35 with part of the genome of the E1 region of an Ad serotype 5 and replacing part of the genome of the E4 region of the Ad serotype 35 with a gene encoding an ORF 4, 6, or 6/7 in the E4 protein of the Ad serotype 5. In this Example, an oncolytic Ad (hereinafter also referred to as "OAd35") described in Patent Literature 1 (WO 2020/166727 A1) was used as Comparative Example (FIG. 1). With regard to the complete sequence of a human Ad serotype 5 genome, reference was made to a sequence registered in GenBank Accession NO: M73260.1.

### 1. Production of OAd35-E1/E4

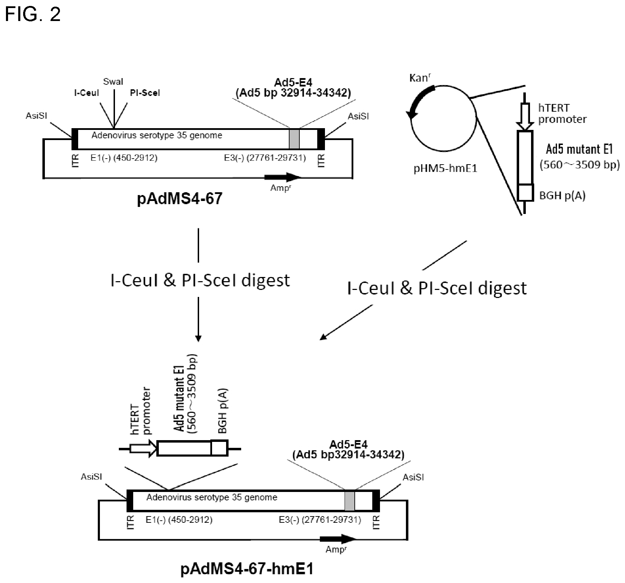

A plasmid (pAdMS4-67-hmE1) for producing the OAd35-E1/E4 was produced as described below. First, a pAdMS4-67 was produced by replacing part of the E4 gene of the Ad serotype 35 with the gene (32,914-34,342 bp of the Ad serotype 5: SEQ ID NO: 1) encoding the ORF 4, 6, or 6/7 in the E4 protein of the Ad serotype 5. A fragment obtained by digesting pHM5-hmE1, a plasmid carrying a modified gene (560-3,509 bp: SEQ ID NO: 2) encoding the E1 region of the Ad serotype 5 downstream of the hTERT promoter, which was located downstream of the 5' ITR of the Ad serotype 35, with I-CeuI and PI-SceI, and the pAdMS4-67 digested with I-CeuI and PI-SceI were ligated to each other to provide the pAdMS4-67-hmE1 (FIG. 2). With regard to the modified portion (823-837 bp) of the E1 gene of the Ad serotype 5, a base sequence before the modification is set forth in SEQ ID NO: 3, and a base sequence after the modification is set forth in SEQ ID NO: 4.
(SEQ ID NO: 1) Gene encoding ORF 4, 6, or 6/7 of E4 Protein of Ad Serotype 5 (32,914-34,342 bp) 
tcacaga accctagtat tcaacctgcc acctccctcc caacacacag agtacacagt
cctttctccccggctggcct taaaaagcat catatcatgg gtaacagaca
tattcttagg tgttatattc cacacggtttcctgtcgagc caaacgctca
tcagtgatat taataaactc cccgggcagc tcacttaagt tcatgtcgct
gtccagctgc tgagccacag gctgctgtcc aacttgcggt tgcttaacgg
gcggcgaaggagaagtccac gcctacatgg gggtagagtc ataatcgtgc
atcaggatag ggcggtggtg ctgcagcagcgcgaataa actgctgccg
ccgccgctcc gtcctgcagg aatacaacat ggcagtggtc
tcctcagcgatgattcgcac cgcccgcagc ataaggcgcc ttgtcctccg
ggcacagcag cgcaccctga tctcacttaaatcagcacag taactgcagc
acagcaccac aatattgttc aaaatcccac agtgcaaggc
gctgtatccaaagctcatgg cggggaccac agaacccacg tggccatcat
accacaagcg caggtagatt aagtggcgacccctcataaa cacgctggac
ataaacatta cctcttttgg catgttgtaa ttcaccacct
 
cccggtaccatataaacctc tgattaaaca tggcgccatc caccaccatc
ctaaaccagc tggccaaaac ctgcccgccggctatacact gcagggaacc
gggactggaa caatgacagt ggagagccca ggactcgtaa
ccatggatcatcatgctcgt catgatatca atgttggcac aacacaggca
cacgtgcata cacttcctca ggattacaagctcctcccgc gttagaacca
tatcccaggg aacaacccat tcctgaatca gcgtaaatcc
cacactgcagggaagacctc gcacgtaact cacgttgtgc attgtcaaag
tgttacattc gggcagcagc ggatgatcctccagtatggt agcgcgggtt
tctgtctcaa aaggaggtag acgatccctactgtacggag tgcgccgaga
caaccgagat cgtgttggtc gtagtgtcat gccaaatgga
acgccggacgtagtcatatt tcctgaagca aaaccaggtg cgggcgtgac
aaacagatct gcgtctccgg tctcgccgcttagatcgctc tgtgtagtag
ttgtagtata tccactctct caaagcatcc aggcgccccc
tggcttcgggttctatgtaa actccttcat gcgccgctgc cctgataaca
tccaccaccg cagaataagc cacacccagccaacctacac attcgttctg
cgagtcacac acgggaggag cgggaagagc tggaagaacc at
(SEQ ID NO: 2) Gene encoding E1 Region of Ad Serotype 5 (560-3,509 bp) 
a tgagacatat tatctgccac ggaggtgtta ttaccgaaga
aatggccgccagtcttttgg accagctgat cgaagaggta ctggctgata
atcttccacc tcctagccattttgaaccac ctacccttca cgaactgtat
gatttagacg tgacggcccc cgaagatcccaacgaggagg cggtttcgca
gatttttccc gactctgtaa tgttggcggt gcaggaaggg attgacttac
tcacttttcc gccggcgccc ggttctccgg agccgcctca cctttcccgg
cagcccgagcagccggagca gagagccttg ggtccggttt ctatgccaaa
 
ccttgtaccggaggtgatcg atcttacctg ccacgaggct ggctttccac
ccagtgacga cgaggatgaa gagggtgaggagtttgtgtt agattatgtg
gagcaccccg ggcacggttg caggtcttgt cattatcacc
ggaggaatacgggggaccca gatattatgt gttcgctttg ctatatgagg
acctgtggca tgtttgtcta cagtaagtgaaaattatggg cagtgggtga
tagagtggtg ggtttggtgt ggtaattttt tttttaattt
ttacagttttgtggtttaaa gaattttgta ttgtgatttt tttaaaaggt
cctgtgtctg aacctgagcc tgagcccgagccagaaccgg agcctgcaag
acctacccgc cgtcctaaaa tggcgcctgc tatcctgaga cgcccgacat
cacctgtgtc tagagaatgc aatagtagta cggatagctg
tgactccggtccttctaaca cacctcctga gatacacccg gtggtcccgc
tgtgccccat taaaccagtt gccgtgagagttggtgggcg tcgccaggct
gtggaatgta tcgaggactt gcttaacgag cctgggcaac
ctttggacttgagctgtaaa cgccccaggc cataaggtgt aaacctgtga
ttgcgtgtgt ggttaacgcc tttgtttgctgaatgagttg atgtaagttt
aataaagggt gagataatgt ttaacttgca tggcgtgtta
aatggggcggggcttaaagg gtatataatg cgccgtgggc taatcttggt
tacatctgac ctcatggagg cttgggagtgtttggaagat ttttctgctg
tgcgtaactt gctggaacag agctctaacagtacctcttg gttttggaggtttctgtggg
gctcatccca ggcaaagtta gtctgcagaa ttaaggaggattacaagtgg
gaatttgaag agcttttgaa atcctgtggt gagctgtttg attctttgaa
tctgggtcaccaggcgcttt tccaagagaa ggtcatcaag actttggatt
tttccacacc ggggcgcgct gcggctgctgttgctttttt gagttttata
aaggataaat ggagcgaaga aacccatctg agcggggggt
acctgctggatttctggcc atgcatctgt ggagagcggt tgtgagacac
 
aagaatcgcc tgctactgtt gtcttccgtccgcccggcga taataccgac
ggaggagcag cagcagcagc aggaggaagccaggcggcgg cggcaggagc
agagcccatg gaacccgaga gccggcctggaccctcggga atgaatgttg
tacaggtggc tgaactgtat ccagaactga gacgcatttt
gacaattacagaggatgggc aggggctaaa gggggtaaag agggagcggg
gggcttgtga ggctacagag gaggctaggaatctagcttt tagcttaatg
accagacacc gtcctgagtg tattactttt caacagatca
aggataattgcgctaatgag cttgatctgc tggcgcagaa gtattccata
gagcagctga ccacttactg gctgcagccaggggatgatt ttgaggaggc
tattagggta tatgcaaagg tggcacttag gccagattgc
aagtacaagatcagcaaact tgtaaatatc aggaattgtt gctacatttc
tgggaacggg gccgaggtgg agatagatacggaggatagg gtggccttta
gatgtagcat gataaatatg tggccggggg tgcttggcat ggacggggtg
gttattatga atgtaaggtt tactggcccc aattttagcggtacggtttt
cctggccaat accaacctta tcctacacgg tgtaagcttc tatgggttta
acaatacctgtgtggaagcc tggaccgatg taaggttcgg gggctgtgcc
ttttactgct gctggaaggg ggtggtgtgtcgccccaaaa gcagggcttc
aattaagaaa tgcctctttg aaaggtgtac cttgggtatc
ctgtctgagggtaactccag ggtgcgccac aatgtggcct ccgactgtgg
ttgcttcatg ctagtgaaaa gcgtggctgtgattaagcat aacatggtat
gtggcaactg cgaggacagg gcctctcaga tgctgacctg
ctcggacggcaactgtcacc tgctgaagac cattcacgta gccagccact
ctcgcaaggcctggccagtg tttgagcata acatactgac ccgctgttcc
ttgcatttgg gtaacaggag gggggtgttcctaccttacc aatgcaattt
gagtcacact aagatattgc ttgagcccga gagcatgtcc
 
aaggtgaacctgaacggggt gtttgacatg accatgaaga tctggaaggt
gctgaggtac gatgagaccc gcaccaggtgcagaccctgc gagtgtggcg
gtaaacatat taggaaccag cctgtgatgc tggatgtgac
cgaggagctgaggcccgatc acttggtgct ggcctgcacc cgcgctgagt
ttggctctag cgatgaagat acagattga
(SEQ ID NO: 3) E1 Gene Portion of Ad Serotype 5 (823-837 bp)
   cttacctg ccacgag
(SEQ ID NO: 4) Modified Portion of E1 Gene of Ad Serotype 5 (823-837 bp)
   gttacctc ccacgat

A plasmid DNA obtained by treating the pAdMS4-67-hmE1 with a restriction enzyme AsiSI (manufactured by New England Biolabs, Inc.) to linearize the plasmid was transfected into HEK293 cells with Lipofectamine^{™} 2000 (Invitrogen) to provide the OAd35-E1/E4. After that, the OAd35-E1/E4 was prepared in a large amount by being caused to tertiarily and quaternarily infect the HEK293 cells. Each of the resultant Ads was purified by cesium chloride density gradient centrifugation, and was dialyzed with a solution formed of 10 mM Tris (pH: 7.5), 1 mM MgCl₂, and 10% glycerol.

### 2. Production of OAd35 serving as Comparative Example

The OAd35 was produced by a method described in Patent Literature 1 (WO 2020/166727 A1).

### (Experimental Example 1) Virus Titer (HEK293 Cells and H1299 Cells)

In this Experimental Example, cells were collected from a total of ten 150 mmΦ dishes in each of which a cytopathic effect (CPE) occurred after each of the OAd35-E1/E4 and the OAd35 produced in Example 1 had been caused to act thereon, and 500 µL of a virus solution of the OAd35 and 300 µL of a virus solution of the OAd35-E1/E4 were obtained. The physical titers (virus particles; VPs) of the respective virus solutions were measured by the method of Maizel et al. (Virology, 36: 115-125 (1968)), and the biological titers (plaque forming units; PFUs) thereof were measured by the method of Kanegae et al. (Jpn J Med Sci Biol, 47, 157-166, 1994) through use of HEK293 cells or H1299 (human non-small cell lung cancer) cells. The measured values are shown in Table 1.

**Table 1**

| | VP/mL | HEK293 Cells | | H1299 Cells | |
|---|---|---|---|---|---|
| | | PFU/mL | VP/PFU ratio | PFU/mL | VP/PFU ratio |
| OAd35 | 8.47×10¹¹ | 9.29×10⁸ | 911 | 1.03×10⁸ | 8,203 |
| OAd35-E1/E4 | 1.00×10¹² | 1.14×10¹¹ | 8.80 | 1.07×10¹⁰ | 93.8 |
| | | | | | |
| Proportion between ratios (Ratio of OAd35)/(ratio of OAd35-E1/E4) | | | 103 | | 87.5 |

As a result, irrespective of which one of the cells the oncolytic Ads were cultured in, each of the physical titer (VPs/mL) and biological titer (PFUs/mL) of the OAd35-E1/E4 showed a value higher than that of the OAd35. Further, the value (ratio) of the physical titer (VPs) to the biological titer (PFUs) shown by the OAd35-E1/E4 was lower than that shown by the OAd35. Specifically, at the time of the culture in the HEK293 cells, the ratio of the OAd35 was 103 times as large as the ratio of the OAd35-E1/E4, and at the time of the culture in the H1299 cells, the ratio of the OAd35 was 87.5 times as large as the ratio of the OAd35-E1/E4. It was recognized from the above-mentioned results that the OAd35-E1/E4 produced viruses each having infectivity to an extremely high degree.

### (Experimental Example 2) Cytocidal Action 1

In this Experimental Example, the cytocidal actions of the OAd35-E1/E4 and the OAd35 produced in Example 1 on HCT116 (human colon cancer) cells, BxPC-3 (human pancreatic cancer) cells, H1299 (human non-small cell lung cancer) cells, and HEK293A (human embryonic kidney) cells were recognized by using a crystal violet method.

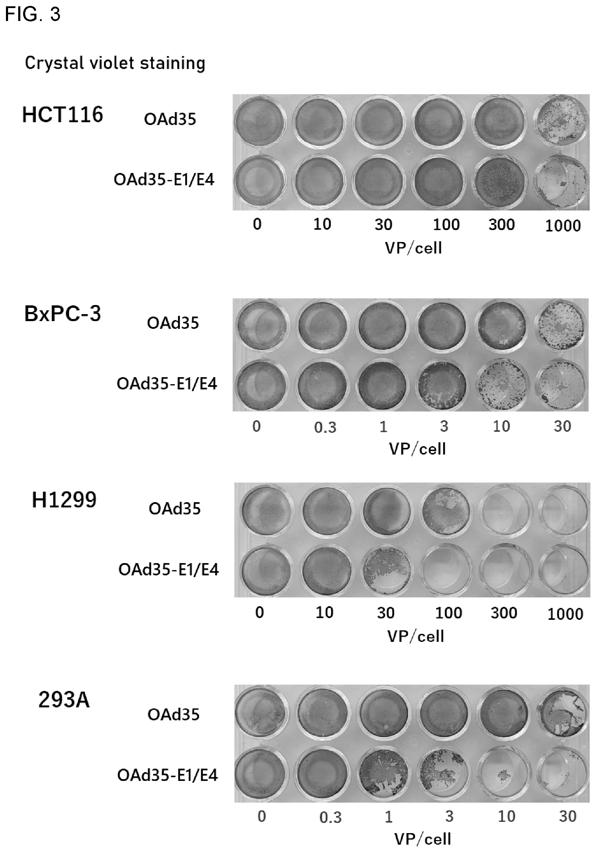

The virus solutions of the OAd35-E1/E4 and the OAd35 having respective concentrations were prepared in the same manner as in Experimental Example 1, and were each added to each of the cells that had been cultured. After the mixture had been cultured at 37°C for 5 days, living adherent cells were fixed with 0.5% glutaraldehyde-containing PBS, and the living cells were stained with 0.1% crystal violet. As a result, a stronger cytocidal effect was recognized when the OAd35-E1/E4 was caused to act on each of the cells (FIG. 3).

### (Experimental Example 3) Cytocidal Action 2

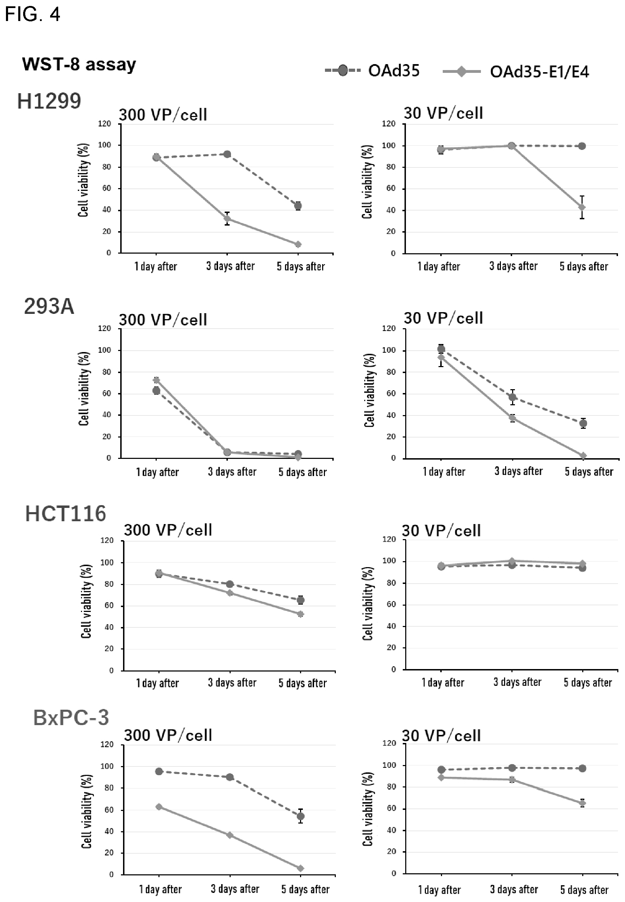

In this Experimental Example, viabilities 1 day, 3 days, and 5 days after each of 300 VP/cell and 30 VP/cell virus solutions of each of the OAd35-E1/E4 and the OAd35 produced in Example 1 had been added to each of HCT116 cells, BxPC-3 cells, H1299 cells, and HEK293A cells, and had been cultured were recognized by a WST-8 assay method (Cell Counting Kit-8, Dojindo Laboratories). As a result, a reduction in cell viability of each of the cells was recognized when the OAd35-E1/E4 was caused to act thereon (FIG. 4).

### (Experimental Example 4) Infection and Growth Potential of Virus

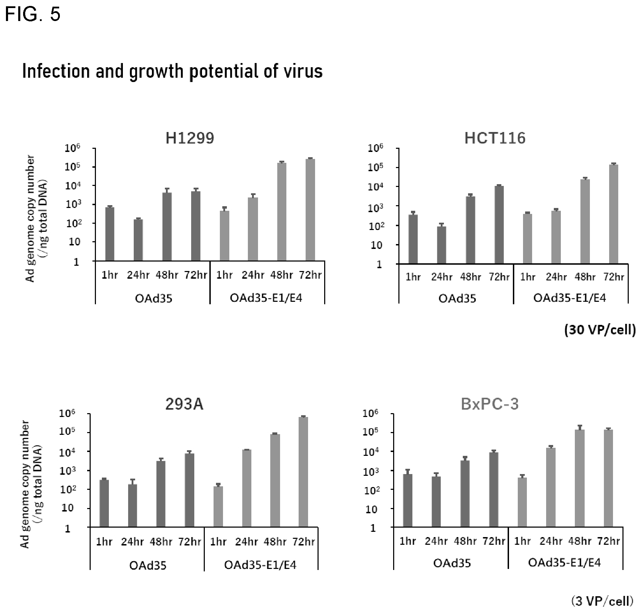

In this Experimental Example, the infection and growth potential of each of the OAd35-E1/E4 and the OAd35 produced in Example 1 when the virus was added at 30 VPs/cell to each of H1299 cells and HCT116 cells, or at 3 VPs/cell to each of HEK293A cells and BxPC-3 cells, and was cultured for up to 72 hours was recognized by measuring a virus genome copy number. The respective cells after their culture for 1 hour, 24 hours, 48 hours, and 72 hours were recovered, and their total DNAs were extracted with DNeasy Blood & Tissue Kit (QIAGEN N.V.), followed by the measurement of their Ad genome copy numbers by a quantitative PCR with THUNDERBIRD SYBR qPCR Mix (Toyobo Co., Ltd.) (FIG. 5). StepOnePlus^{™} real-time PCR systems (Applied Biosystems) were used in the measurement.

The ratios of the infection and growth potentials of each of the OAd35-E1/E4 and the OAd35 when the virus is cultured in each of the cells for 48 hours and 72 hours to that when the virus is cultured therein for 1 hour are shown in Table 2. As a result, it was recognized that as compared to the OAd35, the OAd35-E1/E4 showed a large width of an increase in virus genome copy number, and hence had a high virus growth potential.

**Table 2**

| Infection and growth potential of virus | | | | | |
|---|---|---|---|---|---|
| H1299 | As compared to that 1 hr after culture (x hr/1 hr) | | HCT116 | As compared to that 1 hr after culture (x hr/1 hr) | |
| | 48 hr | 72 hr | | 48 hr | 72 hr |
| OAd35 | 6.05 times | 7.15 times | OAd35 | 8.88 times | 30.94 times |
| OAd35-E1/E4 | 366.94 times | 619.49 times | OAd35-E1/E4 | 62.03 times | 339.95 times |

| 293A | As compared to that 1 hr after culture (x hr/1 hr) | | BxPC-3 | As compared to that 1 hr after culture (x hr/1 hr) | |
|---|---|---|---|---|---|
| | 48 hr | 72 hr | | 48 hr | 72 hr |
| OAd35 | 9.90 times | 24.78 times | OAd35 | 5.38 times | 14.02 times |
| OAd35-E1/E4 | 532.74 times | 4,557.38 times | OAd35-E1/E4 | 354.06 times | 354.12 times |

### (Experimental Example 5) Virus Infectivity

In this Experimental Example, the OAd35-E1/E4 and the OAd35 produced in Example 1 were each evaluated for its virus infectivity by the ratio of its physical titer (VPs/mL) to its biological titer (PFUs/mL). As the number of infectious viruses increases, the numerical value of the ratio reduces.

Each of the viruses OAd35-E1/E4 and OAd35 was scaled up to thirty 150 mmϕ dishes, and each virus was purified by the same approach as that of Example 1. As a physical titer, the concentration (VPs/mL) of virus particles per 1 mL was measured by measuring the absorbance of the virus at 260 nm (UV) in accordance with the method of Maizel et al., Virology, 36: 115-125 (1968). The concentrations (VPs/mL) of the OAd35-E1/E4 and the OAd35 were substantially identical to each other. The number of plaque forming units (PFUs/mL) was measured as the biological titer of the virus on the basis of the TCID₅₀ thereof in 293A cells.

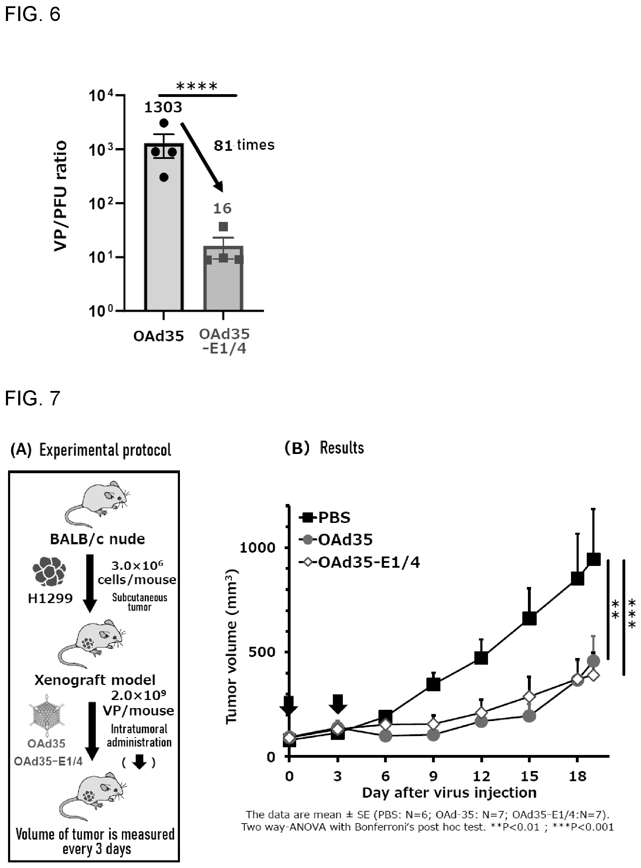

As a result, it was recognized that the virus infectivity of the OAd35-E1/E4 was about 80 times as high as that of the OAd35 (FIG. 6). Thus, the inventors have succeeded in recovering an OAd35 having high infectivity by replacing each of the E1 and E4 regions of an Ad with an Ad serotype 5-derived protein.

### (Experimental Example 6) Tumor Growth-suppressing Action

In this Experimental Example, the tumor growth-suppressing action of each of the OAd35-E1/E4 and the OAd35 produced in Example 1 was recognized in vivo by using a nude mouse.

3×10⁶ H1299 cells (human lung cancer cell line) were subcutaneously implanted into the nude mouse, and the OAd35-E1/E4 or the OAd35 was intratumorally administered at 2×10⁹ VPs thereto on each of Day 0 and Day 3 in accordance with a protocol illustrated in FIG. 7(A). The volume of the tumor was measured every 3 days.

As a result, the OAd35-E1/E4 showed a tumor growth-suppressing action comparable to that of the OAd35 (FIG. 7(B)).

### (Experimental Example 7) Virus Growth Action in Tumor

In this Experimental Example, the virus growth action of each of the OAd35-E1/E4 and the OAd35 produced in Example 1 in a tumor was recognized by using a nude mouse.

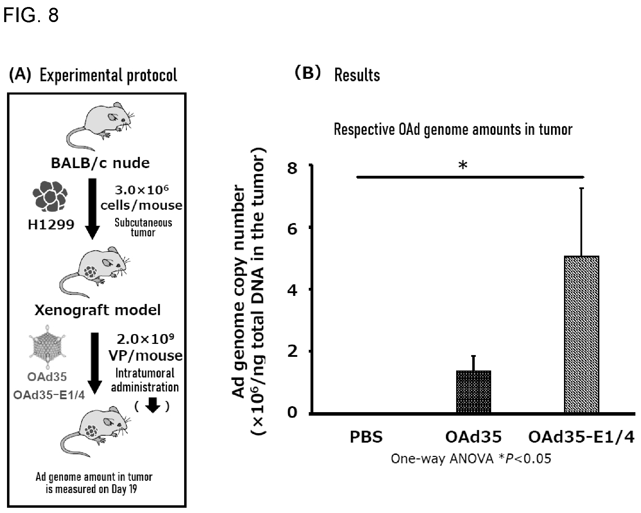

A human lung cancer cell line was subcutaneously implanted into the nude mouse, and each of the OAd35-E1/E4 and the OAd35 was intratumorally administered thereto by the same approach as that of Experimental Example 6 in accordance with a protocol illustrated in FIG. 8(A). The subcutaneous tumor was recovered on Day 19, and the amount of each virus genome in the tumor was measured. The virus genome amount was analyzed by a quantitative PCR method.

As a result, although no statistically significant difference was observed, a large number of virus genome copies was detected in the OAd35-E1/E4 than in the OAd35. Accordingly, it was suggested that the OAd35-E1/E4 showed a high virus growth action even in a tumor (FIG. 8(B)).

### Industrial Applicability

As described above in detail, the recombinant oncolytic Ad of the present invention showed a physical titer and a biological titer higher than those of, for example, a related-art Ad serotype 35, and hence a high virus yield was recognized. Further, the recombinant oncolytic Ad had a high biological titer, and was hence able to secure a certain amount of viruses each having high activity. The Ad serotype except the Ad serotype 5 such as the Ad serotype 35 can eliminate an influence caused by an existing antibody because its anti-Ad antibody retention ratio in a human is extremely low as compared to that of the Ad serotype 5. In addition, CD46 serving as the receptor of the Ad serotype 35, which is expressed in substantially all human cells, is increased in expression particularly in a cancer cell, and hence its infection into the cancer cell can be enhanced. The recombinant oncolytic Ad of the present invention can sufficiently exhibit the antitumor action of an oncolytic Ad.

## Claims

1. A recombinant oncolytic adenovirus, comprising, as a backbone, an adenovirus serotype except an adenovirus serotype 5,
wherein part or an entirety of an E1 gene of the adenovirus serotype 5 is incorporated into an E1 region of a genome of the adenovirus serotype except the adenovirus serotype 5, and
wherein part or an entirety of an E4 gene of the adenovirus serotype 5 is incorporated into an E4 region of the genome of the adenovirus serotype except the adenovirus serotype 5.

2. The recombinant oncolytic adenovirus according to claim 1, wherein the part or the entirety of the E1 gene of the adenovirus serotype 5 includes at least a gene encoding an E1B55kD protein of the adenovirus serotype 5.

3. The recombinant oncolytic adenovirus according to claim 1, wherein the part or the entirety of the E4 gene of the adenovirus serotype 5 includes one or a plurality of kinds of genes selected from a gene encoding an open reading frame (ORF) 4 in an E4 protein of the adenovirus serotype 5, a gene encoding an ORF 6 therein, and a gene encoding an ORF 6/7 therein.

4. The recombinant oncolytic adenovirus according to claim 1, wherein the recombinant oncolytic adenovirus further comprises a tumor-specific promoter incorporated thereinto.

5. The recombinant oncolytic adenovirus according to claim 4, wherein the tumor-specific promoter is any promoter selected from a hTERT promoter, a survivin promoter, an α-fetoprotein promoter, a carcinoembryonic antigen (CEA) promoter, a prostate-specific antigen promoter, a midkine promoter, and a cyclooxygenase-2 (COX-2) promoter.

6. The recombinant oncolytic adenovirus according to claim 1, further comprising a gene encoding a cell death induction-related protein or an immunostimulation-related protein.

7. The recombinant oncolytic adenovirus according to any one of claims 1 to 6, wherein the adenovirus serotype except the adenovirus serotype 5 is an adenovirus serotype 35.

8. A pharmaceutical composition for cancer treatment, comprising the recombinant oncolytic adenovirus of claim 1 as an active ingredient.

9. A reagent for cancer treatment research, comprising the recombinant oncolytic adenovirus of claim 1.

10. A method of producing the recombinant oncolytic adenovirus of claim 1.
